# EUROPÄISCHE PATENTSCHRIFT

(11) **EP 1 052 260 B1**
(45) Veröffentlichungstag und Bekanntmachung des Hinweises auf die Patenterteilung: **07.01.2004**
(21) Anmeldenummer: 00109383.0
(22) Anmeldetag: 02.05.2000
(51) Int. Cl.: C07D 323/06

(54) **Trimerisierung von Formaldehyd in der Gasphase**
Trimerisation of formaldehyde in the gas phase
Trimérisation du formaldéhyd en phase gazeuse

(30) Priorität: 06.05.1999 DE 19920830
(43) Veröffentlichungstag der Anmeldung: 15.11.2000
(73) Patentinhaber: Ticona GmbH, 65451 Kelsterbach (DE)
(72) Erfinder: Werner, Harald, Dr., 61350 Bad Homburg (DE); Schweers, Elke, Dr., 69812 Bad Soden (DE); Olschewski, Frank, Dr., 60529 Unterliederbach (DE); Geiss, Gerhard, 65835 Liederbach (DE); Hufsky, Elfriede, 61191 Rodheim (DE); Tränkler, Helmut, 60320 Frankfurt (DE); Alexander, Heinz, 65929 Frankfurt (DE)

(56) Entgegenhaltungen:
- EP-A- 0 604 884
- EP-A- 0 691 338

## Beschreibung

Die vorliegende Erfindung betrifft ein Verfahren für die Trioxanproduktion aus wasserarmem Formaldehyd in der Gasphase, das dadurch gekennzeichnet ist, daß bei der Trimerisierung von Formaldehyd zu Trioxan ein fester Phosphorsäure-Katalysator zum Einsatz kommt.

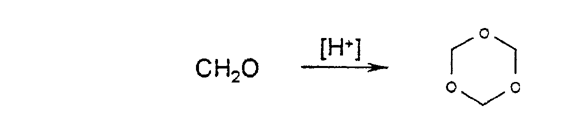

Trioxan stellt das meist verwendete Ausgangsprodukt für die Herstellung von Polyoxymethylen (POM) dar. POM gehört zur Gruppe der Polyacetale und ist ein technisch sehr hochwertiges Polymer mit ausgezeichneten Eigenschaften. Es ist gekennzeichnet durch hohe Festigkeit, Steifigkeit, Zähigkeit (auch bei tiefen Temperaturen) und Formbeständigkeit sowie gute Wärmestandfestigkeit, geringes Wasseraufnahmevermögen, gute elektrische Eigenschaften, günstiges Gleit- u. Verschleißverhalten und gute Verarbeitbarkeit.

Formaldehyd fällt in den gegenwärtig großtechnisch eingesetzten Herstellverfahren (Silberkontaktverfahren bzw. Formoxverfahren) als wäßrige Lösung an. Üblicherweise wird Trioxan daher in der Flüssigphase aus wäßrigen Formaldehydlösungen (Formalin) unter Verwendung von Mineralsäuren (z.B. Schwefelsäure) oder sauren Ionenaustauscherharzen (z.B. sulfoniertes Polystyrol) hergestellt.

Ein Verfahren basierend auf wasserarmem, d.h. gänzlich oder möglichst wasserfreiem Formaldehyd ist von großem ökonomischen Interesse, da erhebliche Kosten, die bei der Herstellung von Trioxan nach dem Stand der Technik aufgrund zahlreicher energieintensiver Destillationsschritte und/oder Extraktionsschritte auftreten, vermieden werden können. Ein derartiges Verfahren zur Herstellung von Trioxan in der Gasphase wurde mangels technischer Zugänglichkeit von wasserarmem Formaldehyd noch nicht in die Praxis umgesetzt.

Die technische Verfügbarkeit von wasserarmem Formaldehyd stellt die Grundlage für Verfahren zur Herstellung von Trioxan in der Gasphase dar. Als mögliches Verfahren wird beispielsweise die nicht-oxidative Dehydrierung von Methanol zu Formaldehyd mit natriumhaltigen Katalysatoren beschrieben (DE 3719055 A1, DD 264209 A1, JP63079850, DE 3920811 A1, JP 02040237 A, JP61130252 A, JP 59048429 A und DE19644188 A1). Als katalytisch aktive Spezies wird unter anderem auch atomares Natrium in der Gasphase vermutet, das nach einem Radikalmechanismus die Dehydrierung von Methanol beschleunigt (S. Ruf, G. Emig, Appl. Catal. A 1997, 161, 19-24).

Bezüglich der Umsetzung wasserarmen Formaldehyds zu Trioxan ist beispielsweise aus ÖS 252913 und SV 209441 die Herstellung von Trioxan aus gasförmigem Formaldehyd, der höchstens noch 10 Gew.-% Wasser enthält, bekannt, wobei eine Lewis-Säure (FeCl₃, ZnCl₂, SnCl₄, BF₃, H₃PO₄, H₂SO₄, Ionenaustauscher, Zeolithe) verwendet wird, die auf einem inerten Trägermaterial (SiO₂, Al₂O₃ oder Holzkohle) aufgebracht ist. Als bester Katalysator erwies sich Silikagel, welches mit 10 Gew.-% Schwefelsäure getränkt war. Die besten Werte für die Umsetzung gasförmigem Formaldehyds (62,5% in Inertgas) wurden bei einer Temperatur von 90°C erhalten. Diese Bedingungen sind aber nach dem heutigen Stand der Kenntnis fernab von technischen Bedingungen: Die Reaktionstemperatur liegt im Bereich der Polymerisationsgrenzen von Formaldehyd und die Formaidehydkonzentration bereits weit entfernt von technisch mit der nicht-oxidativen Dehydrierung und/oder weiteren Prozeßschritten erzielbaren Formaldehydkonzentrationen. Auch ein Wasseranteil im Formaldehyd von 10 Gew.-% wird bei der nicht-oxidativen Dehydrierung weit unterschritten.

Bedeutende technische Nachteile des beschriebenen Katalysatorsystems ergeben sich aus dessen Natur: Katalysatoren, die durch Tränkung von Trägern mit flüssigen Säuren hergestellt werden, weisen eine sehr geringe katalytisch aktive Oberfläche auf, da die Poren des Trägers mit Flüssigkeit gefüllt sind. Die Säure ist außerdem nicht chemisch auf dem Träger verankert und wird aus einem Festbett ausgetragen. Dies führt zu einer fortschreitenden Deaktivierung des Katalysators, sowie zu einer Verunreinigung des Produkts. Weiterhin ergibt sich eine Korrosionsproblematik, die die Verwendung teuerer, korrosionsbeständiger Materialien für den Anlagenbau erfordert.

In EP 604884 A1 ist die Trimerisierung von wasserfreiem Formaldehyd an einem Vanadylphosphat-Hemihydrat-Vollkatalysator mit einer Selektivität von nahe 100% und einer geringen Aktivität (22.1 % des maximal erreichbaren Gleichgewichtsumsatzes, Raum-Zeit-Ausbeute: 35,3 g/lh) beschrieben. Bei guter Selektivität ist die Aktivität des Katalysators damit nur mäßig. Der Katalysator ist als Vollkatalysator durch den hohen Vanadiumanteil und ein aufwendiges Herstellungsverfahren (zwei naßchemische Schritte bis zur Aktivmasse, Trocknen, Verformen, Aktivieren) sehr teuer. Für einen technischen Einsatz ist außerdem die sehr geringe mechanische Stabilität des granulierten Vanadylphosphats ein großer Nachteil.

EP 691338 A beschreibt den Einsatz der Heteropolysäure H₄PVMo₁₁O₄₀ * n H₂O (n = 0 - 32) auf einem inerten Trägermaterial bzw. verpreßt mit einem inerten Füllmaterial für die Trimerisierung von wasserarmem Formaldehyd. Dieser Katalysator zeichnet sich durch eine höhere Aktivität gegenüber dem Vanadylphosphat-Hemihydrat aus (Umsatz nahe dem Gleichgewichtsumsatz). Der Verwendung dieses Katalysators stehen Nachteile, wie eine rasche Deaktivierung und hohe Herstellungskosten, entgegen.

In EP 0691338 A1 wird ein Verfahren zur Herstellung von Trioxan beschrieben, das dadurch gekennzeichnet ist, daß eine Klasse von Heteropolysäuren mit der allgemeinen Zusammensetzung H₃PMoₘWₙO₄₀.* xH₂O ; n, m = 4-8; n + m = 12; x = 0-32 in Form eines Trägerkatalysators eingesetzt wird. So soll ein Katalysator auf Basis von Siliciumcarbid mit der Heteropolysäure H₃PMo₆W₆O₄₀ * xH₂O wasserfreien Formaldehyd bei einer Selektivität von 99% zu Trioxan umsetzen, während 93% des Gleichgewichtsumsatzes erreicht werden (Raum-Zeit-Ausbeute: 85,9 g/lh). Auch hier stehen der Verwendung des Katalysatorsystems die rasche Deaktivierung und der hohe Preis entgegen.

Nachteilig ist auch, daß für den Einsatz von Heteropolysäuren als Katalysator teure Trägermaterialien wie Siliciumcarbid benötigt werden und die Herstellung von geeigneten Heteropolysäuren kostspielig ist, da es sich nicht um kommerziell erhältliche Verbindungen handelt und die Synthese der Heteropolysäuren Schritte beinhaltet, die nicht ohne weiteres in einen größeren Maßstab übertragen werden können (z.B. Etherextraktion).

In nicht veröffentlichten Langzeitversuchen der Anmelderin mit geträgerten Heteropolysäuren stellte sich auch heraus, daß unter technischen Bedingungen (hoher Formaldehydpartialdruck) eine Verwendung dieser Katalysatoren nicht zweckmäßig ist. Es wurde eine Deaktivierung beobachtet, die im besten Fall zu einem Aktivitätsverlust von 80% in wenigen Tagen, in manchen Fällen aber sogar zum völligen Verlust der katalytischen Aktivität führte. Der Grund dafür ist sehr wahrscheinlich eine Überreduktion der redoxaktiven Heteropolysäuren. Regenerationsversuche waren nur teilweise erfolgreich, da auch die mechanische Stabilität der Trägerkatalysatoren im Langzeitversuch deutlich abnahm. Als Folge dessen bildete sich Abrieb, der zum Verstopfen der Testreaktoren führte.

Phosphorsäurekatalysatoren, insbesondere sogenannte Solid-Phosphorous-Acid-Katalysatoren (SPA-Katalysatoren) werden in petrochemischen Prozessen wie der Cumolherstellung aus Benzol und Propen oder bei der Oligomerisierung von Alkenen zu sogenanntem Polygasoline verwendet. Ihr Einsatz für die Trimerisierung von Formaldehyd ist bisher nicht beschrieben. Für kommerzielle Anwendungen werden überwiegend Phosphorsäure-Katalysatoren auf der Basis von Siliciumphosphat eingesetzt.

Aufgabe der vorliegenden Erfindung war es, ein insbesondere auch unter technischen Bedingungen langzeitstabiles Katalysatorsystem für die Gasphasentrimerisierung von wasserarmem Formaldehyd bereitzustellen. Dieses Katalysatorsystem sollte außerdem über eine im Vergleich zum Stand der Technik deutlich verbesserte Standzeit verfügen, da eine ausreichende Langzeitstabilität eine entscheidende Voraussetzung für eine technische Realisierung des Prozesses ist. Für das Standzeitproblem gab es bisher keine Lösung.

Es wurde nun überraschend gefunden, daß SPA-Katalysatoren vorteilhaft für die Gasphasentrimerisierung von Formalaehyd zu Trioxan verwendet werden können, wobei das Standzeitproblem gelöst und somit die Realisierung eines technischen Prozesses ermöglicht wird.

Die Erfindung betrifft daher ein Verfahren zur Herstellung von Trioxan, bei dem gasförmiger, wasserarmer Formaldehyd in einer heterogen katalysierten Gasphasenreaktion unter Einsatz eines festen Phosphorsäurekatalysators trimerisiert wird.

Die erfindungsgemäße Gasphasentrimerisierung von Formaldehyd weist gegenüber dem (bisher noch nicht praktizierten) Stand der Technik eine Reihe von Vorteilen auf. Bei guter Selektivität und Aktivität zeigt das System eine ausgezeichnete Langzeitstabilität. Erfreulicherweise können erfindungsgemäß auch bereits kommerziell verfügbare und kostengünstige Siliciumphosphat-Katalysatoren eingesetzt werden, welche formgebungstechnisch bereits optimiert sind (z.B. für die Cumolherstellung) und deren mechanische Stabilität im frischen Zustand wie auch bei getesteten Ausbauproben gut ist.

Die Erfindung betrifft femer die Verwendung von festen Phosphorsäurekatalysatoren zur Trimerisierung von Formaldehyd im allgemeinen sowie einen integrierten Prozeß zur Herstellung von Trioxan, bestehend aus einer nicht-oxidativen Methanoldehydrierung zur Gewinnung von Formaldehyd, einem optionalen Verfahrensschritt zur Aufreinigung des Formaldehyds, einer Gasphasentrimerisierung des Formaldehyds unter Verwendung eines festen Phosphorsäurekatalysators sowie einer Trennstufe, in der Trioxan von nicht umgesetztem Formaldehyd abgetrennt und das nicht umgesetzte Formaldehyd in die Trimerisierungsstufe rückgeführt wird.

Wasserarmer Formaldehyd im Sinne der Erfindung ist Formaldehyd mit einem Restwassergehalt von 0 bis 5 Gew.-%, vorzugsweise von 0 bis 3 Gew.-%. Vorteilhaft enthält der Formaldehyd möglichst wenig Wasser, d.h. er ist annähernd oder gar vollständig wasserfrei. Der Formaldehyd kann die bei seiner Herstellung üblichen Nebenbestandteile in üblichen Mengen beinhalten. Vorteilhaft wird jedoch durch entsprechende Herstell- oder Aufarbeitungsverfahren ein möglichst geringer Anteil von Nebenkomponenten eingestellt.

Feste Phosphorsäurekatalysatoren (SPA-Katalysatoren) und deren Herstellung sind bereits allgemein bekannt. Neben Siliciumphosphat-Katalysatoren sind auch Borphosphat-, Aluminiumphosphat-, Zirkoniumphosphat-, Titanphosphat- und Zinkphosphat-Katalysatoren mit sauren Eigenschaften beschrieben (DE 2116922; EP0469205 B1; J. Soria, J. E. Iglesias, J. Sanz, *J. Chem. Soc. Faraday Trans. 1993,* 89, 2515-2518; A. Tada, .H. Itoh, *Kitami Kogyo Daigaku Kenkyu Hokoku* 1979, 11, 121-125). In EP0386845 B1 wird außerdem die Modifizierung eines Zirkoniumphosphat-Katalysators mit Organosulfonsäuregruppen beschrieben, mit der eine verbesserte Hochtemperaturstabilität erzielt wird.

Für das erfindungsgemäße Verfahren sind SPA-Katalysatoren allgemein vorteilhaft einsetzbar. Sie können für sich alleine, in Verbindung mit anderen SPA-Katalysatoren sowie als Formkörper, beispielsweise als Granulat, Ring, Tablette, Kugel, Strang, Extrudat etc., als Pulver oder auf einen inerten Träger aufgetragen verwendet werden. Der Katalysator kann auch mit inerten Materialien verdünnt werden, beispielsweise um eine Gradientenschüttung im Reaktor mit den damit verbundenen reaktionstechnischen Vorteilen zu erzielen. Die Verdünnung kann entweder durch Mischen der Katalysatorformkörper mit inerten Formkörpern ähnlicher Größe oder durch Abmischen und Verformen von pulverförmigen Phosphorsäurekatalysatoren mit inerten Pulvern, mit oder ohne Verwendung von Bindemitteln geschehen.

Die erreichbare Aktivität, Selektivität und Stabilität hängt allgemein von der Wahl des Katalysatormaterials, des Trägermaterials, der Porosität (d.h. des Porenvolumens) des Katalysators sowie von den gewählten Reaktionsbedingungen ab, die jeweils zu optimieren sind. Prinzipiell sind die SPA-Katalysatoren ähnlich aktiv und selektiv wie Heteropolysäuren, weisen aber eine deutlich verbesserte Langzeitstabilität auf. Die mechanische Stabilität ist deutlich besser als die der geträgerten Heteropolysäurekatalysatoren.

Bevorzugt werden erfindungsgemäß Siliciumphosphat, Borphosphat, Aluminiumphosphat, Zirkoniumphosphat, Titanphosphat, Zinkphosphat oder Mischungen bzw. Mischverbindungen davon eingesetzt. Besonders bevorzugt ist die Verwendung von Siliciumphosphat.

Die SPA-Katalysatoren reagieren empfindlich auf zu hohe Wassergehalte im Formaldehyd. Es ist daher vorteilhaft, Formaldehyd mit einem Restwassergehalt von weniger als 10000 vpm, vorzugsweise weniger als 5000 vpm zu verwenden. 1 vpm (volume part per million) entspricht dabei 1 Volumenanteil pro 10⁶ Volumenanteile, d.h. 1000 vpm = 0,1 Vol.-%. Vorteilhafte SPA-Katalysatoren sind solche, die gegen einen Restwassergehalt von 10000 vpm, vorzugsweise 5000 vpm unempfindlich sind. Vorteilhaft ist es femer, wenn der SPA-Katalysator durch Temperaturbehandlung und Spülen regeneriert und somit ein Aktivitäts- oder Selektivitätsverlust ausgeglichen werden kann.

Der rechnerische Gehalt an Phosphorsäure im SPA-Katalysator liegt im allgemeinen bei 0,5 bis 99 Gew.-%, vorzugsweise bei 1 bis 90 Gew.-% bezogen auf das Gewicht der fertigen, getrockneten Katalysatormasse.

Die innere Oberfläche des SPA-Katalysators beträgt im allgemeinen 0,001 bis 50 m²/g, vorzugsweise 0,001 bis 20 m²/g. Der SPA-Katalysator weist dabei im allgemeinen ein Porenvolumen von 0,01 bis 1 cm³/g, vorzugsweise von 0,05 bis 0,8 cm³/g auf. Der Anteil von Mikroporen am Porenvolumen beträgt vorteilhaft weniger als 20%, insbesondere weniger als 10%.

Der Phosphorsäurekatalysator kann auch mit inerten Materialien verdünnt werden. Dies ist besonders vorteilhaft, wenn aus reaktionstechnischen Gründen eine Gradientenschüttung im Reaktor gewünscht wird. Die Verdünnung kann durch Mischen von Katalysatorformkörpern mit inerten Formkörpern ähnlicher Größe oder durch Abmischen und Verformen von pulverförmigen Phosphorsäurekatalysatoren mit inerten Pulvern, wahlweise mit oder ohne die Verwendung von Bindemitteln, erfolgen.

Das nach dem erfindungsgemäßen Verfahren hergestellte Trioxan weist vorteilhaft einen Restgehalt an Ameisensäure von weniger als 0,5 Gew.-% und an Ameisensäuremethylester von weniger als 6 Gew.-% auf. Derart niedrige Restgehalte werden durch andere Verfahren gewöhnlich nicht erreicht, so daß vor der Polymerisation zu POM eine entsprechende zusätzliche Reinigung oder Aufkonzentration erforderlich ist.

Das erfindungsgemäße Verfahren eignet sich besonders als Trimerisierungsschritt in einem integrierten Prozeß, aus einer nicht-oxidativen Methanoldehydrierung zur Gewinnung von Formaldehyd, einer erfindungsgemäßen Gasphasentrimerisierung und einer Trennstufe zur Trennung von Trioxan und nicht umgesetztem Formaldehyd besteht, wobei der Formaldehyd vor der Trimerisierung wahlweise gereinigt werden kann und der in der Trennstufe abgetrennte Formaldehyd in die Trimerisierungsstufe zurück geführt wird.

Das erfindungsgemäße Verfahren wird vorteilhaft bei Reaktionstemperaturen von 80 bis 160°C, vorzugsweise bei 90 bis 145°C, einem Eingangspartialdruck des Formaldehyds von 0,5 bis 5 bar absolut, vorzugsweise 0,5 bis 2 bar, und bei Eingangskonzentrationen des Formaldehyds von 1 bis 100 Vol.-%, vorzugsweise 20 bis 100 Vol.-% durchgeführt. Die Reaktion kann wahlweise in Anwesenheit eines inerten Trägergases durchgeführt werden. Als Trägergas ist beispielsweise Stickstoff, Argon oder Kohlendioxid verwendbar. Stickstoff wird bevorzugt verwendet.

Die nachfolgend beschriebenen Beispiele sollen die Erfindung näher erläutern ohne jedoch deren Umfang zu beschränken.

### Reaktortest für die Gasphasentrimerisierung von wasserfreiem Formaldehyd zu Trioxan:

Die Katalysatoren werden in einem Festbett-Rohrreaktor mit 2,9 cm Rohrdurchmesser und 60 cm Länge getestet. Der Reaktor wird von außen mit einer Öl-Mantelheizung temperiert. Es werden 200 ml der Katalysator-Formkörper vorgelegt. Das Reaktorvolumen vor und nach der Katalysatorschüttung wird mit Glaskugeln aufgefüllt. Die Testapparatur wird von einem Prozeßleitsystem gesteuert und kontinuierlich betrieben.

Als Quelle für wasserfreien Formaldehyd dient Trioxan (Polymerisationsqualität) im Gemisch mit Stickstoff (Anteile: 71/29), das in einem vorgeschalteten Reaktor über Vanadylphosphat-Hemihydrat mit einer Selektivität von > 97% katalytisch zu Formaldehyd zersetzt wird.

Die Reaktionsbedingungen bei den Tests sind 108°C Manteltemperatur am Reaktor, 1150 mbar absoluter Druck. Der Eingangsgasstrom setzt sich zusammen aus 88 Vol.-% Formaldehyd und 12 Vol.-% N₂. Eine Vollanalyse des Reaktoraustrags wird on-line direkt am Reaktorausgang mittels eines kalibrierten Gaschromatographen (GC; 3 Säulen-Schaltung) durchgeführt.

Prinzipiell sind die in den folgenden Beispielen angegeben Umsätze und Raum-Zeit-Ausbeuten noch durch geeignete Wahl der Reaktionsparameter optimierbar. So reagiert das Gleichgewicht der untersuchten Reaktion empfindlich auf den Formaldehydpartialdruck und die Temperatur. Die Trimerisierung von Formaldehyd ist eine exotherme Reaktion, die unter starker Volumenabnahme abläuft. Das Reaktionsgleichgewicht sollte sich demnach durch Druckerhöhung und Temperaturerniedrigung auf die Seite von Trioxan verschieben lassen. Diese Möglichkeiten sind aber stark durch die druck- und temperaturabhängigen Polymerisationsgrenzen von Formaldehyd (Bildung von Paraformaldehyd) begrenzt. Um in diesem Sinne eine sichere und reproduzierbare Klassifizierung der Katalysatoren zu gewährleisten, wurden die oben angegebenen Reaktionsparameter gewählt.

Aus den GC-Daten wurden folgende Größen ermittelt: Umsatz U, Trioxan-Selektivität S, Raum-Zeit-Ausbeute *RZA*.

### Beispiel 1

Es wurde ein kommerzieller Siliciumphosphat-Katalysator (C84-5, Fa. Süd-Chemie) ohne weitere Vorbehandlung eingesetzt. Der Katalysator lag als Extrudat (Durchmesser: 5,5 - 7,5 mm, Länge: 8 - 14 mm) vor und enthielt nach Angabe des Herstellers 68 - 80 Gew.-% Säure. Das Porenvolumen betrug 0,19 cm³/g.

Reaktortest: Der Einbau des Katalysators erfolgte wie oben beschrieben. Nach der Temperierung auf 108°C wurde der Reaktor mit dem Formaldehyd-Stickstoff-Gemisch beaufschlagt. Ein konstanter Betriebszustand wurde nach ca. 8h erreicht; insgesamt wurde der Katalysator über 85 h Betriebsdauer untersucht.

| Versuch | Katalysator | Quelle | U % | S % | RZA g/l*h | Desakt. %/24h | Hot Spot °C | Hot Spot Lage |
|---|---|---|---|---|---|---|---|---|
| WETR26, 27 | H₃PO₄/SiO₂ | Süd-Chemie | 41,4 | >96,2 | 82,4 | 0 | 125 | stabil |

### Beispiel 2

Es wurde ein kommerzieller Siliciumphosphat-Katalysator (CA-131, Fa. Süd-Chemie) ohne weitere Vorbehandlung eingesetzt. Der Katalysator lag als Extrudat (Durchmesser: 6 - 7 mm, Länge: 9 - 16 mm) vor und enthielt nach Angabe des Herstellers 81 - 83 Gew. % Säure. Das Porenvolumen betrug 0,23 cm³/g.

Reaktortest: Der Einbau des Katalysators erfolgte wie oben beschrieben. Nach der Temperierung auf 108°C wurde der Reaktor mit dem Formaidehyd-Stickstoff-Gemisch beaufschlagt. Ein konstanter Betriebszustand wurde nach ca. 8h erreicht; insgesamt wurde der Katalysator über 85 h Betriebsdauer untersucht.

| Versuch | Katalysator | Quelle | U % | S % | RZA g/l*h | Desakt. %/24h | Hot Spot °C | Hot Spot Lage |
|---|---|---|---|---|---|---|---|---|
| WETR28 | H₃PO₄/SiO₂ | Süd-Chemie | 39,8 | >95,4 | 78,5 | 0 | 125 | stabil |

### Beispiel 3

Herstellung des Katalysators: 250 ml SiO₂/Al₂O₃-Pellets (ca. 5 mm, Norton SA 5223) wurden mit 30g 85%iger Phosphorsäure so imprägniert, daß 90% des Porenvolumens des Trägermaterials gefüllt waren. Die imprägnierten Pellets wurden anschließend für 15 Minuten durchmischt und dann bei 250°C für eine Stunde calciniert.

Reaktortest: Der Einbau des Katalysators erfolgte wie oben beschrieben. Nach der Temperierung auf 108°C wurde der Reaktor mit dem Formaldehyd-Stickstoff-Gemisch beaufschlagt. Ein konstanter Betriebszustand wurde nach ca. 16h erreicht; insgesamt wurde der Katalysator über 36 h Betriebsdauer untersucht.

| Versuch | Katalysator | Quelle | U¹ % | S¹ % | RZA¹ g/l*h | Deakt. %/24h | Hot Spot¹ °C | Hot Spot Lage |
|---|---|---|---|---|---|---|---|---|
| WETR32 | H₃PO₄/SiO₂/ Al₂O₃ | eigene Herst. | 28,4 | > 98,4 | 58,0 | 19,5 | 125 | stabil |

| | | | | | | | | |
|---|---|---|---|---|---|---|---|---|
| ¹ nach 16 h Betriebsdauer | | | | | | | | |

### Beispiel 4

Herstellunq des Katalysators: 250 ml TiO₂-Ringe (ca. 5x5 mm, Norton XT90045) wurden mit 40,8g 85%iger Phosphorsäure so imprägniert, daß 90% des Porenvolumens des Trägermaterials gefüllt waren. Die imprägnierten Ringe wurden anschließend für 15 Minuten durchmischt und dann bei 250°C für eine Stunde calciniert.

Reaktortest: Der Einbau des Katalysators erfolgte wie oben beschrieben. Nach der Temperierung auf 108°C wurde der Reaktor mit dem Formaldehyd-Stickstoff-Gemisch beaufschlagt. Ein konstanter Betriebszustand wurde nach ca. 6h erreicht; insgesamt wurde der Katalysator über 24 h Betriebsdauer untersucht.

| Versuch | Katalysator | Quelle | U % | S % | RZA g/l*h | Deakt. %/24h | Hot Spot °C | Hot Spot Lage |
|---|---|---|---|---|---|---|---|---|
| WETR30 | H₃PO₄/TiO₂ | eigene Herst. | 39,7 | >97,4 | 80,0 | 0 | 124 | stabil |

### Vergleichsbeispiel 1

Herstellunq des Katalvsators: 250 ml Aluminiumoxidkugeln (ca. 5 mm, Norton SA5262) wurden mittels der Porenfüllmethode mit einer wäßrigen Lösung von 5g H₃PMo₅W₇O₄₀ imprägniert und für 10 Minuten durchmischt. Anschließend wurde die Hauptmenge des Wassers mittels eines Rotationsverdampfers von den imprägnierten Formkörpern abgezogen. Abschließend wurde der Katalysator noch für 12 Stunden bei 150°C getrocknet.

Reaktortest: Der Einbau des Katalysators erfolgte wie oben beschrieben. Nach der Temperierung auf 108°C wurde der Reaktor mit dem Formaldehyd-Stickstoff-Gemisch beaufschlagt. Ein konstanter Betriebszustand wurde selbst nach ca. 100 h nicht erreicht; insgesamt wurde der Katalysator über 100 h Betriebsdauer untersucht.

| Versuch | Katalysator | Quelle | U¹ % | S¹ % | RZA¹ g/l^{*}h | Deakt. %/24h | Hot Spot¹ °C | Hot Spot Lage |
|---|---|---|---|---|---|---|---|---|
| WETR21 | HPA/α-Al₂O₃ | eigene Herst. | 45,0 | >96,7 | 90,0 | 16 | 115 | wandert |

| | | | | | | | | |
|---|---|---|---|---|---|---|---|---|
| ¹ nach 8 h Betriebsdauer | | | | | | | | |

### Vergleichsbeispiel 2

Herstellung des Katalysators: 250 ml Siliciumcarbidringe (5 x 5 mm, Norton SC5532) wurden mittels der Porenfüllmethode mit einer wäßrigen Lösung von 5g H₃PMo₅W₇O₄₀ imprägniert und für 10 Minuten durchmischt. Anschließend wurde die Hauptmenge des Wassers mittels eines Rotationsverdampfers von den imprägnierten Formkörpern abgezogen. Abschließend wurde der Katalysator noch für 12 Stunden bei 150°C getrocknet.

Reaktortest: Der Einbau des Katalysators erfolgte wie oben beschrieben. Nach der Temperierung auf 108°C wurde der Reaktor mit dem Formaldehyd-Stickstoff-Gemisch beaufschlagt. Ein konstanter Betriebszustand wurde nach nicht erreicht; insgesamt wurde der Katalysator über 24 h Betriebsdauer untersucht.

| Versuch | Katalysator | Quelle | U¹ % | S¹ % | RZA¹g/l*h | Deakt. %/24h | Hot Spot¹ °C | Hot Spot Lage |
|---|---|---|---|---|---|---|---|---|
| WETR33 | HPA/SiC | eigene Herst. | 29,9 | 91,2 | 56,7 | 25 | 114 | wandert |

| | | | | | | | | |
|---|---|---|---|---|---|---|---|---|
| ¹ nach 8 h Betriebsdauer | | | | | | | | |

## Patentansprüche

1. Verfahren zur Herstellung von Trioxan, wobei gasförmiger, wasserarmer Formaldehyd in einer heterogen katalysierten Gasphasenreaktion unter Verwendung eines festen Phosphorsäurekatalysators (SPA-Katalysators) trimerisiert wird.

2. Verfahren nach Anspruch 1, **dadurch gekennzeichnet, daß** der verwendete feste Phosphorsäurekatalysator im wesentlichen aus Siliciumphosphat, Borphosphat, Aluminiumphosphat, Zirkoniumphosphat, Titanphosphat, Zinkphosphat oder Mischungen bzw. Mischverbindungen dieser Phosphate besteht.

3. Verfahren nach Anspruch 1, **dadurch gekennzeichnet, daß** der Restwassergehalt des wasserarmen Formaldehyds kleiner als 10000 vpm, vorzugsweise kleiner als 5000 vpm ist.

4. Verfahren nach einem der Ansprüche 1 bis 3, **dadurch gekennzeichnet, daß** ein Phosphorsäurekatalysator eingesetzt wird, der unempfindlich gegen Restwassergehalte des Formaldehyds von kleiner oder gleich 10000 vpm, vorzugsweise kleiner oder gleich 5000 vpm ist.

5. Verfahren nach Anspruch 1 und 2, **dadurch gekennzeichnet, daß** der Phosphorsäurekatalysator bei Aktivitäts- oder Selektivitätsverlust durch Temperaturbehandlung und Spülen mit Stickstoff regeneriert werden kann.

6. Verfahren nach Anspruch 1 und 2, **dadurch gekennzeichnet, daß** es bei einer Reaktionstemperatur im Bereich von 80 bis 160°C, einem Eingangspartialdruck des Formaldehyds von 0,5 bis 5 bar absolut, und einer Eingangskonzentration des Formaldehyds von 1 bis 100% durchgeführt wird.

7. Verfahren nach Anspruch 1, 2 und 6, **dadurch gekennzeichnet, daß** die Reaktion in Anwesenheit eines inerten Trägergases, vorzugsweise Stickstoff, Argon oder Kohlendioxid, durchgeführt wird.

8. Verfahren nach Anspruch 1 und 2, **dadurch gekennzeichnet, daß** der rechnerische Gehalt an Phosphorsäure im Phosphorsäurekatalysator zwischen 0,5 und 99 Gew.-% bezogen auf die fertige Katalysatormasse beträgt.

9. Verfahren nach einem der Ansprüche 1, 2 und 8, **dadurch gekennzeichnet, daß** der Phosphorsäurekatalysator eine innere Oberfläche zwischen 0,001 und 50 m²/g und ein Porenvolumen von 0,01 und 1 cm³/g aufweist.

10. Verfahren nach Anspruch 9, **dadurch gekennzeichnet, daß** der Anteil der Mikroporen am Porenvolumen des verwendeten festen Phosphorsäurekatalysators kleiner 20%, bevorzugt kleiner 10% ist.

11. Verfahren nach Anspruch 1 und 2, **dadurch gekennzeichnet, daß** der Phosphorsäurekatalysator als Formkörper eingesetzt wird.

12. Verfahren nach Anspruch 1, 2 und 11, **dadurch gekennzeichnet, daß** der Phosphorsäurekatalysator mit inerten Materialien verdünnt ist.

13. Verwendung von festen Phosphorsäurekatalysatoren für die Trimerisierung von Formaldehyd.

14. Verwendung des nach einem oder mehreren der voran- oder nachgestellten Ansprüchen hergestellten Trioxans mit Restgehalten an Ameisensäure von weniger als 0,5 Gew.-% und an Ameisensäuremethylester von weniger als 6 Gew.-% direkt oder nach weiterer Aufreinigung für die Herstellung von Oxymethylen-Homo- oder Copolymeren.

15. Integrierter Prozeß, bestehend aus einer nicht-oxidativen Methanoldehydrierung, wahlweise mit oder ohne Aufreinigung des Rohformaldehyds, einer nachfolgenden Gasphasentrimerisierung unter Verwendung eines festen Phosphorsäurekatalysators sowie einer Trennstufe, in der das gewonnene Trioxan von nicht umgesetztem Formaldehyd abgetrennt, und das nicht umgesetzte Formaldehyd in die Trimerisierungsstufe zurück geführt wird.

## Claims

1. A process for preparing trioxane, which comprises trimerizing gaseous formaldehyde having a low water content in a heterogeneously catalyzed gas-phase reaction using a solid phosphoric acid catalyst (SPA catalyst).

2. The process as claimed in claim 1, wherein the solid phosphoric acid catalyst used consists essentially of silicon phosphate, boron phosphate, aluminum phosphate, zirconium phosphate, titanium phosphate, zinc phosphate or a mixture or mixed compounds of these phosphates.

3. The process as claimed in claim 1, wherein the residual water content of the formaldehyde having a low water content is less than 10,000 vpm, preferably less than 5000 vpm.

4. The process as claimed in any one of claims 1 to 3, wherein the phosphoric acid catalyst used is insensitive to residual water contents in the formaldehyde of less than or equal to 10,000 vpm, preferably less than or equal to 5000 vpm.

5. The process as claimed in claim 1 or 2, wherein the phosphoric acid catalyst can be regenerated by thermal treatment and flushing with nitrogen when the activity or selectivity drops.

6. The process as claimed in claim 1 or 2 carried out at a reaction temperature in the range from 80 to 160°C, an inlet partial pressure of formaldehyde of from 0.5 to 5 bar absolute and an inlet concentration of formaldehyde of from 1 to 100%.

7. The process as claimed in claim 1, 2 or 6, wherein the reaction is carried out in the presence of an inert carrier gas, preferably nitrogen, argon or carbon dioxide.

8. The process as claimed in claim 1 or 2, wherein the calculated content of phosphoric acid in the phosphoric acid catalyst is from 0.5 to 99% by weight, based on the finished catalyst composition.

9. The process as claimed in any one of claims 1, 2 and 8, wherein the phosphoric acid catalyst has an internal surface area of from 0.001 to 50 m²/g and a pore volume of from 0.01 to 1 cm³/g,

10. The process as claimed in claim 9, wherein the proportion of the pore volume made up by micropores in the solid phosphoric acid catalyst used is less than 20%, preferably less than 10%.

11. The process as claimed in claim 1 or 2, wherein the phosphoric acid catalyst is used as a shaped body.

12. The process as claimed in claim 1, 2 or 11, wherein the phosphoric acid catalyst is diluted with inert materials.

13. The use of a solid phosphoric acid catalyst for the trimerization of formaldehyde.

14. The use of trioxane prepared as claimed in one or more of the above or following claims and having residual contents of formic acid of less than 0.5% by weight and of methyl formate of less than 6% by weight either directly or after further purification for the preparation of oxymethylene homopolymers or copolymers.

15. An integrated process comprising a nonoxidative methanol dehydrogenation, either with or without purification of the crude formaldehyde, a subsequent gas-phase trimerization using a solid phosphoric acid catalyst and also a separation step in which the trioxane obtained is separated from unreacted formaldehyde and the unreacted formaldehyde is returned to the trimerization step.

## Revendications

1. Procédé de préparation de trioxanne, du formaldéhyde gazeux, pauvre en eau, étant trimérisé dans une réaction en phase gazeuse catalysée par voie hétérogène avec utilisation d'un catalyseur solide à l'acide phosphorique (catalyseur SPA).

2. Procédé selon la revendication 1, **caractérisé en ce que** le catalyseur solide à l'acide phosphorique utilisé se compose pour l'essentiel de phosphate de silicium, de phosphate de bore, de phosphate d'aluminium, de phosphate de zirconium, de phosphate de titane, de phosphate de zinc ou de mélanges, respectivement, de composés mixtes de ces phosphates.

3. Procédé selon la revendication 1, **caractérisé en ce que** la teneur résiduelle en eau du formaldéhyde pauvre en eau est inférieure à 10000 vpm, de préférence inférieure à 5000 vpm.

4. Procédé selon l'une quelconque des revendications 1 à 3, **caractérisé en ce que** l'on utilise un catalyseur à l'acide phosphorique qui est insensible vis-à-vis de teneurs en eau résiduelles du formaldéhyde inférieures ou égales à 10000 vpm, de préférence inférieures ou égales à 5000 vpm.

5. Procédé selon les revendications 1 et 2, **caractérisé en ce que** le catalyseur à l'acide phosphorique peut être régénéré, en cas de perte d'activité ou de sélectivité, à l'aide d'un traitement thermique et d'une purge à l'azote.

6. Procédé selon les revendications 1 et 2, caractérisé en ce qu'il est effectué à une température de réaction dans le domaine de 80 à 160°C, sous une pression partielle d'entrée du formaldéhyde de 0,5 à bars en pression absolue et sous une concentration d'entrée du formaldéhyde de 1 à 100%.

7. Procédé selon les revendications 1, 2 et 6, **caractérisé en ce que** la réaction est effectuée en présence d'un gaz vecteur inerte, de préférence l'azote, l'argon ou le dioxyde de carbone.

8. Procédé selon les revendications 1 et 2, **caractérisé en ce que** la teneur calculée en acide phosphorique dans le catalyseur à l'acide phosphorique se situe entre 0,5 et 99% en poids, par rapport à la masse de catalyseur finie.

9. Procédé selon l'une quelconque des revendications 1, 2 et 8, **caractérisé en ce que** le catalyseur à l'acide phosphorique présente une surface interne comprise entre 0,001 et 50 m²/g et un volume de pores compris entre 0,01 et 1 cm³/g.

10. Procédé selon la revendication 9, **caractérisé en ce que** la proportion de micropores dans le volume de pores du catalyseur solide à l'acide phosphorique utilisé est inférieure à 20%, de préférence inférieure à 10%.

11. Procédé selon les revendications 1 et 2, **caractérisé en ce que** le catalyseur à l'acide phosphorique est utilisé sous la forme d'un corps de forme.

12. Procédé selon les revendications 1, 2 et 11, **caractérisé en ce que** le catalyseur à l'acide phosphorique est dilué à l'aide de matériaux inertes.

13. Utilisation de catalyseurs solides à l'acide phosphorique en vue de la trimérisation du formaldéhyde.

14. Utilisation du trioxanne fabriqué conformément à l'une quelconque ou plusieurs des revendications précédentes ou subséquentes, ayant des teneurs résiduelles en acide formique de moins de 0,5% en poids et en esters méthyliques de l'acide formique de moins de 6% en poids, directement ou après purification ultérieure, en vue de la fabrication d'homopolymères ou de copolymères d'oxyméthylène.

15. Procédé intégré, se composant d'une déshydrogénation au méthanol non oxydante, au choix avec ou sans purification du formaldéhyde brut, d'une trimérisation en phase gazeuse subséquente avec utilisation d'un catalyseur solide à l'acide phosphorique ainsi que d'une étape de séparation, lors de laquelle le trioxanne obtenu est séparé du formaldéhyde n'ayant pas réagi et lors de laquelle le formaldéhyde n'ayant pas réagi est reconduit dans l'étape de trimérisation.
